# FASCICULE DE BREVET EUROPEEN

(11) **EP 1 476 243 B1**
(45) Date de publication et mention de la délivrance du brevet: **30.08.2006**
(21) Numéro de dépôt: 03715071.1
(22) Date de dépôt: 05.02.2003
(51) Int. Cl.: B01D 53/047, C01B 3/56, C07C 7/12

(54) **TRAITEMENT DES MELANGES HYDROGENE/HYDROCARBURES SUR ADSORBANTS REGENERES A HAUTE PRESSION**
BEHANDLUNG VON WASSERSTOFF/KOHLENWASSERSTOFF MISCHUNGEN MIT BEI HOHEM DRUCK REGENERIERTEN ADSORPTIONMITTELN
TREATMENT OF HYDROGEN/HYDROCARBON MIXTURES ON ADSORBENTS REGENERATED AT HIGH PRESSURE

(30) Priorité: 15.02.2002 FR 0201919
(43) Date de publication de la demande: 17.11.2004
(73) Titulaire: L'air Liquide, S.A. à Directoire et Conseil de Surveillance pour l'Etude et l'Exploitation des Procédés Georges Claude, 75321 Paris Cedex 07 (FR)
(72) Inventeur: DE SOUZA, Guillaume, F-92130 Issy les Moulineaux (FR); TROMEUR, Pascal, F-45520 Cercottes (FR)
(74) Mandataire: Pittis, Olivier
(86) Numéro de dépôt international: PCT/FR2003/000350
(87) Numéro de publication internationale: WO 2003/070357

(56) Documents cités:
- EP-A- 1 076 035
- EP-A- 1 095 701
- EP-A- 1 132 341
- WO-A-97/45363
- DE-A- 2 624 346

## Description

La présente invention concerne un procédé PSA de séparation d'un mélange gazeux contenant de l'hydrogène (H₂) et des impuretés de type hydrocarbures (CₙHₘ), dans lequel le mélange gazeux à purifier est mis en contact avec un charbon actif et du gel de silice de manière à adsorber les impuretés contenues dans le mélange gazeux à traiter et à produire un flux riche en hydrogène, et à produire, par ailleurs, un flux de gaz résiduaire à une pression de régénération comprise entre 2 et 10 bar abs.

Le procédé d'adsorption modulée en pression ou PSA (Pressure Swing Adsorption) est très fréquemmènt utilisé pour la séparation et la purification des gaz.

Dans le cas du traitement de gaz riches en hydrogène, ce procédé permet de générer un flux d'hydrogène pur, typiquement la pureté excède 99% en volume, et un gaz résiduaire appauvri en hydrogène concentrant les autres espèces présentes dans le mélange gazeux initial à purifier.

Deux étapes principales caractérisent tout procédé PSA, à savoir :
- une phase d'adsorption durant laquelle le gaz de charge est mis en contact avec un ou plusieurs lits contenant chacun un ou plusieurs adsorbants, à une pression d'adsorption (P) à laquelle les composés autres que l'hydrogène sont adsorbés et donc retenus sur le (ou les) adsorbant solide. Le gaz sortant du lit est l'hydrogène purifié qui est produit à une pression de production (P') proche de la pression d'adsorption (P) ; généralement, la différence entre ces pressions P et P' est inférieure à 1 bar.
- une phase de désorption pendant laquelle le (ou les) adsorbants est balayé par un gaz d'élution autre que le gaz de charge à une pression de régénération (P"), telle que : P"<P, à laquelle les composés adsorbés sont désorbés, puis récupérés en aval du lit d'adsorbant à cette pression de régénération (P"). A l'issue de cette étape de désorption, l'adsorbant peut subir une nouvelle étape d'adsorption.

Plus la pression de régénération (P") est basse, plus la désorption des composés indésirables est efficace. Cette pression de régénération (P") a donc un fort impact sur la pureté de l'hydrogène produit, sur le taux de récupération d'hydrogène et sur la quantité d'adsorbant nécessaire.

En pratique, les adsorbants généralement utilisés pour le traitement des mélanges H₂/hydrocarbures nécessitent une pression de régénération (P") comprise entre 1.5 et 2 bar abs, mais toujours inférieure à 3 bar abs. En effet, si la pression de régénération excède cette valeur maximale, les espèces plus lourdes que le propane qui sont toujours présentes dans le flux de gaz à purifier s'adsorbent de façon définitive sur l'adsorbant et l'empoisonnent rapidement.

Un tel procédé est, par exemple, décrit dans la demande DE-A-2624346.

Par ailleurs, sur tous les sites pétrochimiques, les gaz résiduaires riches en hydrocarbures de toutes les unités et en particulier ceux des unités de purification d'hydrogène ne peuvent être relâchés à l'atmosphère. Ils sont collectés sur un réseau de gaz combustible qui alimente les différents brûleurs du site.

La pression de ce réseau (P"') est généralement comprise entre 4 et 7 bar abs, c'est-à-dire en pratique toujours supérieure à 3 bar abs.

On comprend donc que, de par la différence de pression existante, les gaz résiduaires d'unités PSA ne peuvent jamais être dirigés directement vers le réseau combustible d'un site pétrochimique.

Pour tenter de résoudre ce problème, plusieurs solutions ont déjà été proposées.

Selon une solution connue, le gaz résiduaire sortant de l'unité PSA est comprimé par une machine tournante, tel un compresseur de gaz, permettant de faire passer la pression de ce gaz résiduaire de la pression P" à la pression P"' de manière à pouvoir ensuite l'introduire dans le réseau combustible du site pétrochimique.

Selon une autre solution connue, les brûleurs de plusieurs fours sont changés pour permettre la combustion du gaz à la pression P", ce qui évite de devoir le comprimer, comme dans le cas précédent.

Toutefois, ces deux solutions connues sont très coûteuses puisqu'elles multiplient, en général, par un facteur de 1.5 à 3 le coût global de la purification de l'hydrogène.

Le problème qui se pose alors est de pouvoir purifier économiquement des gaz riches en hydrogène, en particulier ceux contenant au moins une espèce hydrocarbure lourd du type C₃+, c'est-à-dire dont le nombre d'atomes de carbone est supérieur ou égal à 3, et ce, sans se heurter aux problèmes rencontrés dans l'art antérieur.

La solution de l'invention est alors un procédé PSA de séparation d'un mélange gazeux contenant de l'hydrogène (H₂) et des impuretés choisies parmi les hydrocarbures (CₙHₘ), dans lequel :
a) on met le mélange gazeux à purifier en contact avec un premier adsorbant contenant au moins un gel de silice et avec un deuxième adsorbant contenant au moins un charbon actif de manière à adsorber sur lesdits adsorbants au moins une partie des impuretés contenues dans le mélange gazeux à traiter,
b) on produit de l'hydrogène à une pression de production (P'),
c) on désorbe au moins une partie des impuretés adsorbées à l'étape (a),
(d) on produit un flux de gaz résiduaire contenant lesdites impuretés désorbées à l'étape (c), ledit flux de gaz résiduaire étant produit à une pression de régénération (P") comprise entre 2 et 10 bar abs.

Selon le cas, le procédé de l'invention peut comprendre l'une ou plusieurs des caractéristiques techniques suivantes :
- la pression de régénération (P") est supérieure ou égale à 3 bar abs.
- la pression de régénération (P") est supérieure ou égale à 4 bar abs.
- le flux de gaz résiduaire est produit directement à ladite pression de régénération (P") sans étape préalable de compression de ce gaz résiduaire.
- le gaz à l'étape (a) est à une pression d'adsorption (P) comprise entre 20 et 50 bar abs.
- préalablement à l'étape (a), on met le mélange gazeux à purifier en contact avec un adsorbant de type alumine activée.
- l'alumine activée a une aire massique d'au moins 200 m²/g, de préférence supérieure à 270 m²/g, et un volume poreux supérieur à 0.25 cm³/g.
- le gel de silice a une aire massique supérieure à 400 m²/g, de préférence supérieure à 600 m²/g, et un volume poreux supérieur à 0.25 cm³/g.
- le charbon actif a une aire massique supérieure à 600 m²/g, de préférence supérieure à 850 m²/g, et un volume poreux supérieur à 0.25 cm³/g.
- à l'étape b), on produit de l'hydrogène à une pression de production (P') comprise entre 20 et 50 bar abs et/ou à une pureté d'au moins 98.5 % vol.
- le flux de gaz résiduaire produit à l'étape d) alimente un réseau combustible d'un site pétrochimique, en particulier une raffinerie.
- le flux de gaz résiduaire produit à l'étape d) alimente une canalisation dans laquelle circule un gaz ou mélange gazeux à une pression comprise entre 3 et 8 bar abs.
- le mélange gazeux à purifier contient, en outre, des impuretés choisies parmi le CO₂, la vapeur d'eau, N2 et CO.
- le mélange gazeux à purifier contient des hydrocarbures à au moins 3 atomes de carbone dans leur chaîne hydrocarbonée, de préférence des hydrocarbures choisis parmi le propane, propène, butane, butène, pentane, hexane, benzène, toluène, xylène et leurs isomères.
- le mélange gazeux à purifier est mis en contact d'abord avec le gel de silice puis avec le charbon actif.
- le mélange gazeux à purifier est issu d'une unité de réformage catalytique.

L'invention permet grâce à l'utilisation d'une combinaison particulière de plusieurs lits d'adsorbants d'opérer l'étape de régénération à haute pression et, plus précisément, à une pression supérieure ou égale à celle du réseau combustible du site pétrochimique sur lequel est installée l'unité PSA.

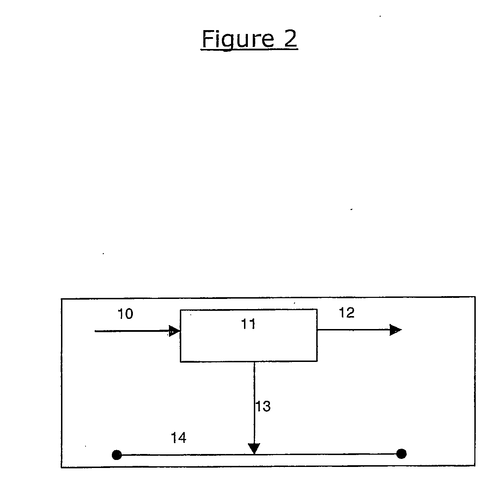

Ainsi, la figure 2 illustre l'intégration de ce procédé PSA sur un site pétrochimique. Le gaz de charge à traiter 10 est introduit dans l'unité PSA 11 pour y être purifié par le procédé de l'invention et récupérer ainsi un flux d'hydrogène purifié 12. Par ailleurs, le gaz résiduaire 13 est également récupéré et peut être envoyé, selon l'invention, sous une pression régénération comprise entre 3 et 10 bar abs par exemple, dans le réseau 14 de gaz combustible du site pétrochimique.

L'invention permet une augmentation significative de la pression de régénération de l'adsorbant en procédé PSA et permet donc ainsi d'éviter l'étape très fréquente et très coûteuse de compression du gaz résiduaire en sortie du PSA.

Les quantités respectives de charbon et de gel de silice sont choisies en fonction de la teneur en C₃+ du gaz de charge, de la pression P du gaz de charge, de la pression P" du gaz résiduaire et de la pureté souhaitée de l'hydrogène produit.

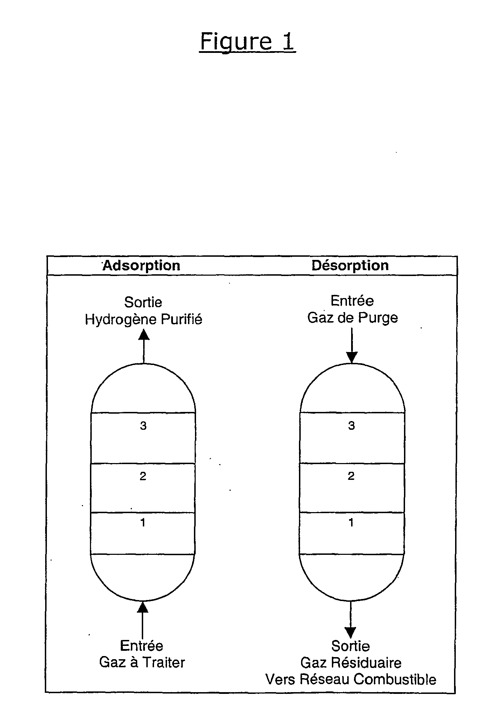

De préférence, l'adsorbant de l'invention comporte un lit d'alumine activée en entrée, lequel est suivi d'un lit de gel de silice et d'un lit de charbon actif dans les proportions données ci-après et ainsi qu'illustré sur la figure ci-jointe. En effet, comme on le voit sur la figure 1 :
- la couche d'alumine 1, située du côté alimentation (en entrée) de la zone d'adsorption, représente de 0 à 10 % en volume de la quantité totale d'adsorbant,
- la couche de gel de silice 2, prise en sandwich entre les couches d'alumine 1 et de charbon actif 3, représente de 30 à 70 % de la quantité totale d'adsorbant et
- la couche de charbon actif 3, située du côté production (en sortie) de la zone d'adsorption, représente de 30 à 70% de la quantité totale d'adsorbant.

Le gaz à purifier traverse donc successivement la couche d'alumine 1, la couche de gel de silice 2 et la couche de charbon actif 3.

L'aire massique de l'alumine activée utilisée doit être d'au moins 200 m²/g, de préférence supérieure à 270 m²/g, avec un volume poreux supérieur à 0.25 cm³/g. Elle peut être éventuellement dopée avec un composé alcalin susceptible d'adsorber réversiblement le gaz carbonique (CO₂). La taille des billes est comprise entre 1 et 5 mm, de préférence entre 2 et 3.5 mm.

Le gel de silice mis en oeuvre a une aire massique supérieure à 400 m²/g, de préférence supérieure à 600 m²/g, et un volume poreux supérieur à 0.25 cm³/g. La taille des billes est comprise entre 1 et 5 mm, de préférence entre 2 et 3.5 mm. Ce gel de silice présente une constante de Henry au moins deux fois moindre que celle du charbon actif auquel il est associé et dont les propriétés sont les suivantes :

Le charbon actif a une aire massique supérieure à 600 m²/g , de préférence supérieure à 850 m²/g, et un volume poreux supérieur à 0.25 cm³/g. La taille des particules doit être comprise entre 1 et 5 mm.

Le procédé de l'invention peut être mis en oeuvre dans des unités PSA utilisant de 3 à 20 adsorbeurs, de préférence de l'ordre de 6 à 12.

### Exemple

L'efficacité du procédé de l'invention a été vérifiée lors de la purification d'un flux d'hydrogène issu d'une unité de réformage catalytique située sur un site de raffinage, dont la composition et les caractéristiques sont données dans le tableau I ci-après.

**Tableau I**

| **Gaz de Charge** | **Caractéristiques** |
|---|---|
| Pression d'adsorption | 26 bar abs |
| Température | 40°C |
| Constituants | Composition molaire |
| H₂ | 75% |
| CH₄ | 11% |
| C₂H₆ | 7% |
| C₃H₈ | 4% |
| C₄H₁₀ | 2.5% |
| C₅+ | 0.5% |

Le flux gazeux a été soumis à une purification par voie PSA au moyen d'une combinaison d'adsorbants selon l'invention et, à titre comparatif, une succession d'adsorbants selon l'art antérieur (charbon seul)

Les conditions de fonctionnement de l'unité PSA classique étaient les suivantes :
- pression d'adsorption : 26 bar abs
- nombre d'adsorbeurs : 6
- nombre équilibrages : 3
- température du gaz : 40°C
- gel de silice : commercialisé par Engelhard sous la référence Sorbead
- charbon actif : commercialisé par Norit sous la référence R3 Extra
- proportion gel de silice / charbon : 50/50 (0/100 selon l'art antérieur)

Les résultats obtenus (pureté, taux de récupération) sont donnés dans le tableau II ci-dessous.

**Tableau II**

| | Adsorbants Conventionnels Charbon | | Adsorbants de l'invention 50% Gel de silice + 50% Charbon | |
|---|---|---|---|---|
| Pression de régénération | 1.6 bar abs | 6 bar abs | 1.6 bar abs | 6 bar abs |
| Pureté de l'hydrogène produit, %vol | 99.5% | Opération Impossible. | 99.5 | 99.5 |
| Taux de récupération de l'hydrogène dans le produit pur | 90% | Désorption incomplète des composés C₃+ | 89% | 65% |
| Débit d'H₂ Produit par volume d'adsorbant | 688 Nm³/h/m³ | | 656 Nm³/h/m³ | 142 Nm³/h/m³ |

Comme il ressort du tableau II, seule la combinaison judicieuse d'adsorbants selon l'invention permet de produire de l'hydrogène à une pureté de 99.5 % tout en produisant le gaz résiduaire sous une pression de 6 bar abs sans étape de compression Dans le cas d'un charbon selon l'art antérieur, la désorption des hydrocarbures lourds (C3+) est incomplète. L'adsorbant est très rapidement contaminé par les impuretés contenues dans le gaz d'alimentation, ce qui l'empêche de maintenir au cours du temps une pureté de produit supérieure à 99.5%.

## Revendications

1. Procédé PSA de séparation d'un mélange gazeux contenant de l'hydrogène (H₂) et des impuretés choisies parmi les hydrocarbures (CₙHₘ), dans lequel :
a) on met le mélange gazeux à purifier en contact avec un premier adsorbant contenant au moins un gel de silice et avec un deuxième adsorbant contenant au moins un charbon actif de manière à adsorber sur lesdits adsorbants au moins une partie des impuretés contenues dans le mélange gazeux à traiter,
b) on produit de l'hydrogène à une pression de production (P'),
c) on désorbe au moins une partie des impuretés adsorbées à l'étape (a),
(d) on produit un flux de gaz résiduaire contenant lesdites impuretés désorbées à l'étape (c), ledit flux de gaz résiduaire étant produit à une pression de régénération (P") comprise entre 2 et 10 bar abs.

2. Procédé selon la revendication 1, **caractérisé en ce que** la pression de régénération (P") est supérieure ou égale à 3 bar abs.

3. Procédé selon la revendication 1, **caractérisé en ce que** la pression de régénération (P") est supérieure ou égale à 4 bar abs.

4. Procédé selon l'une des revendications 1 à 3, **caractérisé en ce que** le flux de gaz résiduaire est produit directement à ladite pression de régénération (P") sans étape préalable de compression de ce gaz résiduaire.

5. Procédé selon l'une des revendications 1 à 4, **caractérisé en ce que** le gaz à l'étape (a) est à une pression d'adsorption (P) comprise entre 20 et 50 bar abs.

6. Procédé selon l'une des revendications 1 à 5, **caractérisé en ce que**, préalablement à l'étape (a), on met le mélange gazeux à purifier en contact avec un adsorbant de type alumine activée.

7. Procédé selon l'une des revendications 1 à 6, **caractérisé en ce que** l'alumine activée a une aire massique d'au moins 200 m²/g, de préférence supérieure à 270 m²/g, et un volume poreux supérieur à 0.25 cm³/g.

8. Procédé selon l'une des revendications 1 à 7, **caractérisé en ce que** le gel de silice a une aire massique supérieure à 400 m²/g, de préférence supérieure à 600 m²/g, et un volume poreux supérieur à 0.25 cm³/g.

9. Procédé selon l'une des revendications 1 à 8, **caractérisé en ce que** le charbon actif a une aire massique supérieure à 600 m²/g, de préférence supérieure à 850 m²/g, et un volume poreux supérieur à 0.25 cm³/g.

10. Procédé selon l'une des revendications 1 à 9, **caractérisé en ce qu'**à l'étape b), on produit de l'hydrogène à une pression de production (P') comprise entre 20 et 50 bar abs et/ou à une pureté d'au moins 98.5 % vol.

11. Procédé selon l'une des revendications 1 à 10, **caractérisé en ce que** le flux de gaz résiduaire produit à l'étape d) alimente un réseau combustible d'un site pétrochimique, en particulier une raffinerie.

12. Procédé selon l'une des revendications 1 à 11, **caractérisé en ce que** le flux de gaz résiduaire produit à l'étape d) alimente une canalisation dans laquelle circule un gaz ou mélange gazeux à une pression comprise entre 3 et 8 bar abs.

13. Procédé selon l'une des revendications 1 à 12, **caractérisé en ce que** le mélange gazeux à purifier contient, en outre, des impuretés choisies parmi le CO₂, la vapeur d'eau, N2 et CO.

14. Procédé selon l'une des revendications 1 à 13, **caractérisé en ce que** le mélange gazeux à purifier contient des hydrocarbures à au moins 3 atomes de carbone dans leur chaîne hydrocarbonée, de préférence des hydrocarbures choisis parmi le propane, propène, butane, butène, pentane, hexane, benzène, toluène, xylène et leurs isomères.

15. Procédé selon l'une des revendications 1 à 14, **caractérisé en ce que** le mélange gazeux à purifier est mis en contact d'abord avec le gel de silice puis avec le charbon actif.

16. Procédé selon l'une des revendications 1 à 15, **caractérisé en ce que** le mélange gazeux à purifier est issu d'une unité de réformage catalytique.

## Claims

1. PSA method for separating a gas mixture containing hydrogen (H₂) and impurities selected among the hydrocarbons (CₙHₘ), in which:
a) the gas mixture to be purified is contacted with a first adsorbent containing at least a silica gel and with a second adsorbent containing at least an activated charcoal in order to adsorb on said adsorbents at least a portion of the impurities present in the gas mixture to be treated,
b) hydrogen is produced at a production pressure (P'),
c) at least a portion of the impurities adsorbed in step (a) is desorbed,
d) a waste gas stream is produced containing said impurities desorbed in step (c), said waste gas stream being produced at a regeneration pressure (P") between 2 and 10 bar absolute.

2. Method according to Claim 1, **characterized in that** the regeneration pressure (P") is 3 bar absolute or more.

3. Method according to Claim 1, **characterized in that** the regeneration pressure (P") is 4 bar absolute or more.

4. Method according to one of Claims 1 to 3, **characterized in that** the waste gas stream is produced directly at said regeneration pressure (P") without a prior step of compression of this waste gas.

5. Method according to one of Claims 1 to 4, **characterized in that** the gas in step (a) is at an adsorption pressure (P) between 20 and 50 bar absolute.

6. Method according to one, of Claims 1 to 5, **characterized in that**, prior to step (a), the gas mixture to be purified is contacted with an activated alumina type adsorbent.

7. Method according to one of Claims 1 to 6, **characterized in that** the activated alumina has a surface area per unit mass of at least 200 m²/g, preferably above 270 m²/g, and a pore volume above 0.25 cm³/g .

8. Method according to one of Claims 1 to 7, **characterized in that** the silica gel has a surface area per unit mass above 400 m²/g, preferably above 600 m²/g, and a pore volume above 0.25 cm³/g.

9. Method according to one of Claims 1 to 8, **characterized in that** the activated charcoal has a surface area per unit mass above 600 m²/g, preferably above 850 m²/g, and a pore volume above 0.25 cm³/g.

10. Method according to one of Claims 1 to 9, **characterized in that** in step b), hydrogen is produced at a production pressure (P') between 20 and 50 bar absolute and/or with a purity of at least 98.5 vol %.

11. Method according to one of Claims 1 to 10, **characterized in that** the waste gas stream produced in step d) is sent to a fuel network of a petrochemical facility, particularly a refinery.

12. Method according to one of Claims 1 to 11, **characterized in that** the waste gas stream produced in step d) is sent to a line in which a gas or gas mixture flows at a pressure between 3 and 8 bar absolute.

13. Method according to one of Claims 1 to 12, **characterized in that** the gas mixture to be purified contains, in addition, impurities selected among CO₂, water vapour, N2 and CO.

14. Method according to one of Claims 1 to 13, **characterized in that** the gas mixture to be purified contains hydrocarbons with at least 3 carbon atoms in their hydrocarbon chain, preferably hydrocarbons selected among propane, propene, butane, butene, pentane, hexane, benzene, toluene, xylene and their isomers.

15. Method according to one of Claims 1 , to 14, **characterized in that** the gas mixture to be purified is first contacted with the silica gel and then with the activated charcoal.

16. Method according to one of Claims 1 to 15, **characterized in that** the gas mixture to be purified is produced by a catalytic reforming unit.

## Patentansprüche

1. PSA-Verfahren zur Trennung eines Wasserstoff (H₂) und unter Kohlenwasserstoffen (CₙHₘ) ausgewählte Verunreinigungen enthaltenden Gasgemischs, bei dem man:
a) das zu reinigende Gasgemisch mit einem ersten Adosoprtionsmittel, das mindestens ein Kieselgel enthält, und mit einem zweiten Adsorptionsmittel, das mindestens eine Aktivkohle enthält, in Berührung bringt, so daß an den Adsorptionsmitteln mindestens ein Teil der in dem zu behandelnden Gasgemisch enthaltenen Verunreingigungen adsorbiert wird,
b) Wasserstoff bei einem Produktionsdruck (P') produziert,
c) mindestens einen Teil der in Schritt (a) adsorbierten Verunreinigungen desorbiert,
d) einen Restgasstrom produziert, der die in Schritt (c) desorbierten Verunreinigungen enthält, wobei der Restgasstrom bei einem Regenerationsdruck (P") zwischen 2 und 10 bar abs. produziert wird.

2. Verfahren nach Anspruch 1, **dadurch gekennzeichnet, daß** der Regenerationsdruck (P") größer gleich 3 bar abs. ist.

3. Verfahren nach Anspruch 1, **dadurch gekennzeichnet, daß** der Regenerationsdruck (P") größer gleich 4 bar abs. ist.

4. Verfahren nach einem der Ansprüche 1 bis 3, **dadurch gekennzeichnet, daß** der Restgasstrom ohne vorherige Verdichtung dieses Restgases direkt bei dem Regenerationsdruck (P") produziert wird.

5. Verfahren nach einem der Ansprüche 1 bis 4, **dadurch gekennzeichnet, daß** das Gas in Schritt (a) bei einem Adsorptionsdruck zwischen 20 und 50 bar abs. vorliegt.

6. Verfahren nach einem der Ansprüche 1 bis 5, **dadurch gekennzeichnet, daß** man vor Schritt (a) das zu reinigende Gasgemisch mit einem Adsorptionsmittel vom Typ aktiviertes Aluminiumoxid in Berührung bringt.

7. Verfahren nach einem der Ansprüche 1 bis 6, **dadurch gekennzeichnet, daß** das aktivierte Aluminiumoxid eine spezifische Oberfläche von mindestens 200 m²/g, vorzugsweise mehr als 270 m²/g, und ein Porenvolumen von mehr als 0,25 cm³/g aufweist.

8. Verfahren nach einem der Ansprüche 1 bis 7, **dadurch gekennzeichnet, daß** das Kieselgel eine spezifische Oberfläche von mehr als 400 m²/g, vorzugsweise mehr als 600 m²/g, und ein Porenvolumen von mehr als 0,25 cm³/g aufweist.

9. Verfahren nach einem der Ansprüche 1 bis 8, **dadurch gekennzeichnet, daß** die Aktivkohle eine spezifische Ober.fläche von mehr als 600 m²/g, vorzugsweise mehr als 850 m²/g, und ein Porenvolumen von mehr als 0,25 cm³/g aufweist.

10. Verfahren nach einem der Ansprüche 1 bis 9, **dadurch gekennzeichnet, daß** man in Schritt (b) den Wasserstoff bei einem Produktionsdruck (P') zwischen 20 und 50 bar abs. und/oder mit einer Reinheit von mindestens 98,5 Vol.-% produziert.

11. Verfahren nach einem der Ansprüche 1 bis 10, **dadurch gekennzeichnet, daß** der in Schritt (d) produzierte Restgasstrom einem Brennstoffnetz eines Petrochemiestandorts, insbesondere einer Raffinerie, zugeführt wird.

12. Verfahren nach einem der Ansprüche 1 bis 11, **dadurch gekennzeichnet, daß** der in Schritt (d) produzierte Restgasstrom einer Rohrleitung zugeführt wird, in der ein Gas oder Gasgemisch bei einem Druck zwischen 3 und 8 bar abs. strömt.

13. Verfahren nach einem der Ansprüche 1 bis 12, **dadurch gekennzeichnet, daß** das zu reinigende Gasgemisch außerdem unter CO₂, Wasserdampf, N₂ und CO ausgewählte Verunreinigungen enthält.

14. Verfahren nach einem der Ansprüche 1 bis 13, **dadurch gekennzeichnet, daß** das zu reinigende Gasgemisch Kohlenwasserstoffe mit mindestens 3 Kohlenstoffatomen in der Kohlenwasserstoffkette und vorzugsweise unter Propan, Propen, Butan, Buten, Pentan, Hexan, Benzol, Toluol, Xylol und Isomeren davon ausgewählte Kohlenwasserstoffe enthält.

15. Verfahren nach einem der Ansprüche 1 bis 14, **dadurch gekennzeichnet, daß** man das zu reinigende Gasgemisch zunächst mit dem Kieselgel und dann mit der Aktivkohle in Berührung bringt.

16. Verfahren nach einem der Ansprüche 1 bis 15, **dadurch gekennzeichnet, daß** das zu reinigende Gasgemisch aus einer Einheit zur katalytischen Reformierung stammt.
